# EUROPEAN PATENT SPECIFICATION

(11) **EP 2 387 392 B1**
(45) Date of publication and mention of the grant of the patent: **20.02.2013**
(21) Application number: 08875909.7
(22) Date of filing: 30.12.2008
(51) Int. Cl.: A61K 9/14, A61K 9/20, A61K 31/415

(54) **PHARMACEUTICAL FORMULATIONS OF OLMESARTAN**
PHARMAZEUTISCHE FORMULIERUNGEN VON OLMESARTAN
FORMULATIONS PHARMACEUTIQUES D'OLMÉSARTAN

(43) Date of publication of application: 23.11.2011
(73) Proprietor: Abdi Ibrahim Ilac Sanayi ve Ticaret Anonim Sirketi, Istanbul (TR)
(72) Inventor: FARSHI, Farhad, Bahcesehir 34555 Istanbul (TR); AVCI, Recep, Bahcesehir 34555 Istanbul (TR); SOYLEMEZ, Serdar, Bahcesehir 34555 Istanbul (TR); KOC, Fikret, Bahcesehir 34555 Istanbul (TR); NARSISOGLU, Nadin, Bahcesehir 34555 Istanbul (TR)
(86) International application number: PCT/IB2008/055572
(87) International publication number: WO 2010/076596

(56) References cited:
- EP-A- 1 604 664
- WO-A-2006/029057
- WO-A-2008/149160
- US-A1- 2006 281 800
- US-A1- 2007 160 665

## Description

The present invention is related to pharmaceutical compositions, which have good flowability properties, comprising olmesartan medoxomil or combination of olmesartan medoxomil and hydrochlorothiazide and a lubricant or mixtures of lubricants and a pharmaceutically acceptable excipient or excipients.

Degradations of products in pharmaceuticals are important during drug development, since such degradation shall jeopardise efficacy and safety of the pharmaceuticals. Therefore if degradations could be minimum level, saftey will be better. Additionally, if impurities arising from degradation are minimum level, shelf life of product is extended and it means that pecuniary advantage to the manufacturers and consumers along with parts of supply chain.

Olmesartan medoxomil impurity is olmesartan which is called as OL (olmesartan) in an article (Journal of Pharmaceutical and Biomedical Analysis,47 (2008) 553-559, "Identification of a degradation product in stressed tablets of olmesartan medoxomil by the complementary use of HPLC hyphenated techniques", Murakami et al).

Olmesartan medoxomil is described in EP 0503785 patent.

In Benicar ® and Benicar HCT ® tablets, which are products of Daiichi Sankyo, magnesium stearate is used as a excipient. EP1336407 patent discloses magnesium stearate, calcium stearate, stearic acid etc. are used as a lubricant. However this patent does not disclose effects of lubricants on reducing or preventing impurities arising from degradation.

Additionally, EP1336407 patent does not disclose which lubricant is more suitable to obtain eligible impurity results. Some of the lubricants are more suitable than other ones to reduce and to prevent impurities in pharmaceutical formulations including olmesartan c. Every lubricant does not have the character to prevent or to reduce impurities in same level.

WO 2007 128478 discloses using of stearic acid to achieve stabilization. However stearic acid has bad flowability properties and brings about many problems in industrial scale.

However as a lubricant magnesium stearate is not duly lubricant to prevent impurities since in comparison it is invented that some lubricants entail eligible results rather than magnesium stearate. As a lubricant stearic acid prevents olmesartan acid impurity rather than magnesium stearate. But stearic acid does not provide good flowability as much as magnesium stearate.

It is discovered that some lubricants, have improving effect on stability of olmesartan medoxomil and combination of olmesartan modexomil and hydrochlorothiazide. On the other hand, all of the lubricants do not provide same flowability properties to the pharmaceutical powders and lubricants should provide both preventing impurities and providing good flowability. It is also discovered that some lubricants have good flowability properties along with superior effect on stability.

According to this invention lubricants are selected from group of calcium stearate, sodium stearyl fumarate and zinc or mixtures thereof.

According to this invention tablet core of pharmaceutical composition may include other excipients, but are not limited to , fillers such as lactose monohydrate and microcrystalline cellulose, disintegrant such as hydroxypropyl methyl cellulose , binder such as polyvinylpyrrolidone adherent such as colloidal silica. Coating agent of tablet core may be Opadry ® II Pink.

The pharmaceutical composition comprising from 65 % to 85 % filler, from 8 % to 20 active pharmaceutical ingredient or active pharmaceutical ingredients, from 0.2 % to 0.5 % adherent, from 1 % to 5 % lubricant, from 1 % to 3 % binder, from 4 % to 7 % disintegrant by total weight of the tablet core.

Pharmaceutical composition is preferably oral dosage form. Oral dosage form is preferably tablet. To prepare tablets conventional wet granulation methods may be applied. In wet granulation isopropyl alcohol, but not limited, could be used as wetting agent.

### Example 1

### [Table 1]

[Table]

**Table 1 :Olmesartan Medoxomil Formulation**

| **Ingredients** | **Amount (mg)** |
|---|---|
| Olmesartan Medoxomil | 40 |
| Lactose Monohydrate | 242 |
| Povidone K 30 | 6 |
| Hydroxpropylcellulose | 24 |
| Microcrystalline Cellulose | 80 |
| Colloidal Silica | 1 |
| Calcium Stearate | 4 |

### Example 2

Five tablet formulations below are prepared with lubricants respectively magnesium stearate,calcium stearate,zinc,sodium stearyl Fumarate and stearic acid.

### [Table 2]

[Table]

**Table 2 : Tablet Formulation With Magnesium Stearate**

| **Ingredients** | **Amount (mg)** |
|---|---|
| Olmesartan Medoxomil | 40 |
| Hydrochlorothiazide | 25 |
| Lactose Monohydrate | 255 |
| Povidone K 30 | 8 |
| Hydroxypropylcellulose | 26 |
| Microcrystalline Cellulose | 61 |
| Colloidal Silica | 1 |
| Magnesium Stearate | 4 |

### [Table 3]

[Table]

**Table 3 : Tablet Formulation With Calcium Stearate**

| **Ingredients** | **Amount (mg)** |
|---|---|
| Olmesartan Medoxomil | 40 |
| Hydrochlorothiazide | 25 |
| Lactose Monohydrate | 255 |
| Povidone K 30 | 8 |
| Hydroxypropylcellulose | 26 |
| Microcrystalline Cellulose | 61 |
| Colloidal Silica | 1 |
| Calcium Stearate | 4 |

### [Table 4]

[Table]

**Table 4 : Tablet Formulation With Zinc**

| **Ingredients** | **Amount (mg)** |
|---|---|
| Olmesartan Medoxomil | 40 |
| Hydrochlorothiazide | 25 |
| Lactose Monohydrate | 255 |
| Povidone K 30 | 8 |
| Hydroxypropylcellulose | 26 |
| Microcrystalline Cellulose | 61 |
| Colloidal Silica | 1 |
| Zinc | 4 |

### [Table 5]

[Table]

**Table 5 : Tablet Formulation With Sodium Stearyl Fumarate**

| **Ingredients** | **Amount (mg)** |
|---|---|
| Olmesartan Medoxomil | 40 |
| Hydrochlorothiazide | 25 |
| Lactose Monohydrate | 255 |
| Povidone K 30 | 8 |
| Hydroxypropylcellulose | 26 |
| Microcrystalline Cellulose | 61 |
| Colloidal Silica | 1 |
| Sodium Stearyl Fumarate | 4 |

### [Table 6]

[Table]

**Table 6 : Tablet Formulation With Stearic Acid**

| **Ingredients** | **Amount (mg)** |
|---|---|
| Olmesartan Medoxomil | 40 |
| Hydrochlorothiazide | 25 |
| Lactose Monohydrate | 255 |
| Povidone K 30 | 8 |
| Hydroxypropylcellulose | 26 |
| Microcrystalline Cellulose | 61 |
| Colloidal Silica | 1 |
| Stearic Acid | 4 |

Prepared tablets are stored at 40 ° C and 75 % relative humidity during one week. Impurity results are below.

### [Table 7]

[Table]

**Table 7 : Comparison of Stability and Flowability**

| **Olmesartan Acid Impurity (40 ° C and 75 % Relative Humidity)** | | | | | |
|---|---|---|---|---|---|
| **Lubricant** | **Magnesium Stearate** | **Calcium Stearate** | **Zinc** | **Sodium Stearyl Fumarate** | **Stearic Acid** |
| **Starting Amount** | 0.2 | 0.2 | 0.2 | 0.2 | 0.2 |
| **1 Week** | 0.52 | 0.25 | 0.24 | 0.24 | 0.24 |
| **Properties** | Stability result is not eligible but has flowability in industrial scale | **Stability result is eligible and has flowability in industrial scale** | **Stability result is eligible and has flowability in industrial scale** | **Stability result is eligible and has flowability in industrial scale** | Stability result is eligible but has not flowability in industrial scale |

## Claims

1. A pharmaceutical tablet composition, having improved stability with low impurities and eligible flowability, comprising ;
Olmesartan medoxomil or
Combination of olmesartan medoxornil and hydrochlorothiazide and
A lubricant or lubricants and
A pharmaceutically acceptable excipient or excipients and
A coating agent or coating agents of tablet core
**characterized in that** lubricant is selected from group of calcium stearate, zinc and sodium stearyl fumarate or mixtures thereof.

2. A pharmaceutical composition according to claim 1, pharmaceutical composition comprising from 65 % to 85 % filler, from 8 % to 20 % active pharmaceutical ingredient or combination of active pharmaceutical ingredients, from 0.2 % to 0.5 % adherent, from 1 % to 5 % lubricant, from 1 % to 3 % binder, from 4 % to 7 % disintegrant by total weight of the tablet core.

3. A pharmaceutical composition according claim 2 , filler is lactose monohydrate or microsrystalline cellulose or mixtures thereof

4. A pharmaceutical composition according to claim 2 , adherent is colloidal silica

5. A pharmaceutical composition according to claim 2 , binder is povidone K 30

6. A pharmaceutical composition according to claim 2 , disintegrant is hyroxypropylcellulose

7. A pharmaceutical composition according to claim 1, coating agent of tablet core is Opadry ® II Pink

## Patentansprüche

1. : Eine pharmazeutische Tablet Zusammensetzung mit niedrigen Verunreinigungen und geeignete Fliessfaehigkeit umfasst,
Olmesartan Medoxomil oder
Olmesartan Medoxomil und Hydrochlorothiazid Kombination und
Eine Schmiermittel oder Schmiermitteln und
Eine pharmazeutisch akzeptable Exzipient oder Exzipiente und
Eine Beschichtungsagent oder Beschichtungsagente von Tablettenkern
**Dadurch gekennzeichnet dass** Schmiermittel aus der Gruppe von Calcium stearat, Zink und
Natrium stearyl fumarat oder Mischungen davon gewaehlt ist.

2. : Eine pharmazeutische Zusammensetzung nach Anspruch 1,wobei pharmazeutische Zusammensetzung von 65% bis %85 Füllstoff, von 8% bis 20% pharmazeutisch aktiver Inhaltsstoff oder Kombination von pharmazeutisch aktiven Inhaltsstoffen , von 0.2% bis 0.5% Gleitmittel, von 1% bis 5% Schmiermittel, von 1% bis 3% Bindemittel, von 4% bis 7% Desintegrator bei gesamt Masse von Tablettenkern umfasst.

3. : Eine pharmazeutische Zusammensetzung nach Anspruch 2, wobei Füllstoff Laktose monohydrat oder mikrokristalin Zelluloseoder Mischungen davon ist.

4. : Eine pharmazeutische Zusammensetzung nach Anspruch 2, wobei Gleitmittel koloidales Siliziumdioxid ist.

5. : Eine pharmazeutische Zusammensetzung nach Anspruch 2, wobei Bindemittel Povidon K30 ist.

6. : Eine pharmazeutische Zusammensetzung nach Anspruch 2, wobei Disintegrator Hydroxypropylzellulose ist.

7. : Eine pharmazeutische Zusammensetzung nach Anspruch 1, wobei Beschichtungsmittel von Tablettenkern Opadry ® II Pink ist.

## Revendications

1. Une composition pharmaceutique du comprimé présentant une stabilité améliorée avec un bas niveau d'impureté et une fluidité admissible, contenant:
L'olmésartan médoxomil ou
la combination d'olmésartan médoxomil avec d'hydrochlorothiazide et
Un lubrifiant ou des lubrifiants et
Un excipient ou des excipients pharmaceutiquement acceptables et
Un agent ou des agents d'enrobage pour le noyau du comprimé;
La particularité caractéristique est ce que le lubrifiant est choisi dans le group du stéarate de calcium, du zinc et du stéaryl fumarate de sodium ou de leurs mélanges.

2. Une composition pharmaceutique, selon la revendication 1, composition pharmaceutique contenant du diluant de 65% à 85%, de substance active ou une combination des substances actives de 8% à 20%, d'adhérent de 0,2% à 0,5%, de lubrifiant de 1% à 5%, de liant de 1% à 3%, de désintégrant de 4% à 7% sur la masse totale du noyau du comprimé.

3. Une composition pharmaceutique, selon la revendication 2, où le diluant est le lactose monohydraté ou la cellulose microcrystalline ou bien de leurs mélanges.

4. Une composition pharmaceutique, selon la revendication 2, où l'adhérent est la silice collaïdale.

5. Une composition pharmaceutique, selon la revendication 2, où le liant est la povidon K30.

6. Une composition pharmaceutique, selon la revendication 2, où le désintégrant est la hydroxypropylcellulose.

7. Une composition pharmaceutique, selon la revendication 1, où l'agent d'enrobage du noyau du comprimé est Opadry ® II rose.
